(19) **Europäisches Patentamt**
**European Patent Office**
**Office européen des brevets**

(11) **EP 1 539 128 B1**

(12) **EUROPÄISCHE PATENTSCHRIFT**

(45) Veröffentlichungstag und Bekanntmachung des Hinweises auf die Patenterteilung:
**17.09.2008 Patentblatt 2008/38**

(21) Anmeldenummer: **03757807.7**

(22) Anmeldetag: **09.09.2003**

(51) Int Cl.:
*A61K 31/198* (2006.01)      *A61K 31/401* (2006.01)
*A61K 31/185* (2006.01)      *A61P 17/00* (2006.01)

(86) Internationale Anmeldenummer:
**PCT/EP2003/009985**

(87) Internationale Veröffentlichungsnummer:
**WO 2004/026295 (01.04.2004 Gazette 2004/14)**

(54) **VERWENDUNG VON SUBSTANZEN ZUM SCHUTZ DER HAUT**

USE OF SKIN-PROTECTING SUBSTANCES

UTILISATION DE SUBSTANCES POUR PROTEGER LA PEAU

(84) Benannte Vertragsstaaten:
**AT BE BG CH CY CZ DE DK EE ES FI FR GB GR HU IE IT LI LU MC NL PT RO SE SI SK TR**

(30) Priorität: **17.09.2002 DE 10243233**

(43) Veröffentlichungstag der Anmeldung:
**15.06.2005 Patentblatt 2005/24**

(73) Patentinhaber: **Phenion GmbH & Co. KG**
**40225 Düsseldorf (DE)**

(72) Erfinder:
• **GASSENMEIER, Thomas**
**40229 Düsseldorf (DE)**
• **GRAF, Rüdiger**
**64853 Otzberg (DE)**
• **KOCK, Michael**
**60487 Frankfurt (DE)**
• **BERND, August**
**55411 Bingen/Kempten (DE)**
• **KAUFMANN, Roland**
**63073 Offenbach (DE)**
• **KIPPENBERGER, Stefan**
**60313 Frankfurt (DE)**
• **SCHUBERT-ZSILAVECZ, Manfred**
**61352 Bad Homburg (DE)**
• **STEINHILBER, Dieter**
**61389 Schmitten (DE)**
• **WERZ, Oliver**
**61118 Bad Vilbel (DE)**

(74) Vertreter: **Ulbrich, Carola**
**Henkel AG & Co. KGaA**
**VTP Patente**
**40191 Düsseldorf (DE)**

(56) Entgegenhaltungen:
WO-A-00/04870      WO-A-00/37071
WO-A-01/19330      WO-A-88/09657
WO-A-91/12798      WO-A-03/075903
DE-A- 2 703 964      US-A- 6 114 337

• **PATENT ABSTRACTS OF JAPAN Bd. 1997, Nr. 06, 30. Juni 1997 (1997-06-30) & JP 09 048720 A (SHISEIDO CO LTD), 18. Februar 1997 (1997-02-18)**
• **JANEKE GUIDO ET AL: "Role of taurine accumulation in keratinocyte hydration." JOURNAL OF INVESTIGATIVE DERMATOLOGY, Bd. 121, Nr. 2, August 2003 (2003-08), Seiten 354-361, XP002281082 ISSN: 0022-202X (ISSN print)**
• **DATABASE BIOSIS [Online] BIOSCIENCES INFORMATION SERVICE, PHILADELPHIA, PA, US; September 1997 (1997-09), YANG SUNG-CHIL ET AL: "Effects of taurine on glutamate-induced neurotoxicity and interleukin-6 mRNA expression in astrocytes" XP002281083 Database accession no. PREV199800231610 & KOREAN JOURNAL OF BIOLOGICAL SCIENCES, Bd. 1, Nr. 3, September 1997 (1997-09), Seiten 467-473, ISSN: 1226-5071**

**Beschreibung**

Die Erfindung betrifft die Verwendung mindestens einer Substanz, die ein Zwitterion ausbilden kann, und/oder ihrer Derivate zum Schutz der Haut und/oder der Hautanhangsgebilde.

Die Plasmamembran fast aller tierischer und pflanzlicher Zellen ist hochdurchlässig für Wasser. Besteht ein Ungleichgewicht in der Teilchenkonzentration innerhalb und außerhalb der Zelle, setzt die Osmose ein. Liegt extrazellulär eine höhere Konzentration an gelösten Teilchen vor, kommt es zu einem Wasserausstrom aus der Zelle, die dadurch schrumpft. Eine Erhöhung der intrazellulären Osmolarität führt dagegen zu einem verstärkten Wassereinstrom, der zur Schwellung der Zelle führt.

Unter physiologischen Bedingungen sind die meisten Zellen, insbesondere die meisten Säugetierzellen, kaum schwankenden Osmolaritäten ausgesetzt. Dabei bilden allerdings Zellen des Darms und der Niere, wie beispielsweise Darmepithelzellen, Zellen des Nierenmarks und die Blutzellen in den Kapillaren von Darm und Niere, eine Ausnahme. Diese Zellen schützen sich vor einer Erhöhung der extrazellulären Osmolarität durch die Aufnahme von anorganischen und organischen Osmolyten. Anorganische Osmolyte sind insbesondere die Ionen $Na^+, K^+$ und $Cl^-$. Die wichtigsten organischen Osmolyten in Säugetierzellen sind die Polyole wie z. B. Sorbitol und myo-Inositol.

Die Haut ist die Oberfläche des menschlichen Körpers und ein Organ, das in verschiedenen Schichten aufgebaut ist. Die äußerste dieser Schichten wird von der Epidermis gebildet. Die oberste Schicht der Epidermis wiederum ist das *Stratum Comeum* das aus abgestorbenen Hornzellen besteht. Die Epidermis schützt das darunter liegende Gewebe vor äußeren physikalischen Einflüssen, wie beispielsweise Hitze oder UV-Strahlung, sowie vor chemischen und biologischen Einflüssen, wie beispielsweise Mikroorganismen.

Trockene, spröde, schuppige, empfindliche oder irritierte Haut ist häufig rau oder rissig und kann der Schutzfunktion der intakten Haut nicht vollständig nachkommen. Ein erhöhter transepidermaler Wasserverlust der rissigen bzw. rauen Haut kann neben dem Verlust der Feuchtigkeit und einer damit verbundenen geringeren Elastizität auch zur Erhöhung der Osmolarität im extrazellulären Bereich der Oberhaut führen.

Aufgabe der vorliegenden Erfindung ist es daher, Wirkstoffe zum Schutz der Haut zu finden, die die negativen-Folgen von osmotischem Stress, in der Haut beheben können.

Die WO00/04870 beschreibt den Einsatz einer Zusammensetzung mit verzweigt-kettigen Aminosäuren zur Steigerung der Produktion von epidermalen Lipiden. Glycin kann als weitere Komponente in diesen Zusammensetzungen enthalten sein.

Die WO 01/19330 offenbart die Verwendung von Panthotensäure und/oder ihren Derivaten zur Behandlung von solchen Hautkrankheiten, die mit dem Degranulationsprozess von Mastozyten (wie Allergien oder Psoriasis), verbunden sind. Es wird weiterhin die Verwendung einer Mischung aus Panthotensäure und Glycin zur Herstellung einer entsprechenden medizinischen und/oder kosmetischen Zusammensetzung offenbart.

Die DE 2703964 offenbart die orale oder topische Zufügung von Glycin bei gestörtem Glycin-Stoffwechsel zur Behandlung von Psoriasis.

EP124091 beschreibt eine pharmazeutische Zusammensetzung enthaltend Glycin zur Behandlung von Epilepsie.

WO01/01932 offenbaren Oligosaccharidaldonsäuren zur topischen Anwendung, die auch Glycin enthalten können.

Aufgabe der vorliegenden Erfindung ist es, Wirkstoffe zum Schutz der Haut zu finden, die die negativen Folgen von osmotischem Stress in der Haut beheben können. Eine weitere Aufgabe der vorliegenden Erfindung ist es, einen Wirkstoff zum Schutz der Haut bereitzustellen, der besser an den Wirkort in der Haut vordringen kann als die im Stand der Technik beschriebenen Wirkstoffe.

Unter Haut und Hautanhangsgebilde im erfindungsgemäßen Zusammenhang sind die Haut, die Schleimhaut, die Haare und ihre Haarfollikel, Drüsen und Nägel, insbesondere Haut, Schleimhaut, Haare und Haarfollikel, zu verstehen.

Überraschenderweise wurde gefunden, dass Ethylglycinat in der Lage ist, die Haut und vor allem die Hautzellen, insbesondere die Keratinozyten, vor dem Einfluss von osmolytischem Stress zu schützen.

In der Haut vorkommende Keratinozyten reagieren auf hyperosmolare extrazelluläre Bedingungen mit einer Verringerung der Proliferationsrate (vgl. Abb. 1)

Die Zugabe bereits geringer Mengen von Ethylglycinat kann den proliferationshemmenden Einfluss von hyperosmolaren Stress abschwächen (vgl. Abb. 5 ).

Überraschenderweise wurde darüber hinaus gefunden, dass die am häufigsten verwendeten organischen Osmolyte, die Polyole, wie z. B. Glycerin und Inositol, nicht in der Lage sind, die Auswirkungen des hyperosmolaren Stresses auf die Proliferation von Keratinozyten zu vermindern (Abb. 3 und 4).

Geladene Substanzen werden bevorzugt in Form ihrer Salze mit einem physiologisch verträglichen Gegenion eingesetzt.

Als Mischsalze von positiv geladenen Substraten eignen sich beispielsweise anorganischen Mischsalze wie Hydrochloride, Hydrobromide, Hydroiodide, Sulfate, Sulfite, Hydrogensutfate, Hydrogensulfite, Carbonate und Hydrogencarbonate, Mono-, Di-, Triphosphate oder Mischungen der Phosphate sowie weitere Gemische oder Mischsalze mit organischen Carbonsäuren wie beispielsweise Citronensäure, Milchsäure, Benzoesäure oder Weinsäure.

Ethylglycinat hat den Vorteil, dass der Ester durch Hydrolyse, insbesondere in saurem oder basischem Medium, oder durch auf und/oder in der Haut befindliche Enzyme, insbesondere Hydrolasen, gespalten werden kann.

Ethylglycinat hat weiker den vorteil, dass sich bei der Esterspaltung Abbauprodukte ergeben, die einen positiven Nutzen oder zumindest keine negativen Auswirkungen auf die Haut und/oder die Hautzellen haben.

Gemäß einer bevorzugten Form der vorliegenden Erfindung wird Ethylglycinat zum Schutz der Haut, Hautanhangsgebilden und/oder der Hautzellen vor osmotischem Stress eingesetzt.

Der osmotische Stress kann je nach Osmolarität des extrazellulären Milieus bewirken, dass die Zellen Wasser nach außen abgeben (hyperosmolarer Stress) oder von außen aufnehmen (hypoosmolarer Stress). Eine zu starke Veränderung des Zellvolumens durch Wasseraus- bzw. Wassereinstrom ist im Hinblick auf die Aufrechterhaltung der Zellfunktion und der zelleigenen Prozesse aber unbedingt zu verhindern.

Es konnte beispielsweise gezeigt werden, dass hyperosmolarer Stress auf Keratinozyten und insbesondere auf ihre Proliferationsrate einen starken Einfluss ausübt. Eine mögliche Folge des osmolaren Stresses ist eine frühzeitige Alterung der Haut, die durch die Verwendung von Substanzen mit osmoprotektiver Wirkung vermieden oder zumindest verringert werden kann.

Ein weiterer Gegenstand der Erfindung ist die Verwendung von Ethylglycinat zum Schutz der Haut vor Entzündungen bzw. entzündlichen Reaktionen.

Insbesondere kann Etylglycinat eingesetzt werden, um die Haut vor Entzündungen, welche von osmotischem Stress verursacht werden und somit eine weitere Folge des osmotischen Stresses darstellen, zu schützen. Die Entzündungsreaktionen können bspw. durch die erhöhte Expression von Interleukin 6 (IL-6) nachgewiesen werden. Auch gegen die Überexpression von IL-6 in Keratinozyten schützt die Zugabe der erfindungsgemäßen Substanzen (vgl. Beispiele).

Ein weiterer Gegenstand der Erfindung ist die Verwendung von Ethylglycinat zum Schutz der Haut vor UV-Stress. UV-Stress wird durch Bestrahlung der Haut mit ultraviolettem Licht hervorgerufen. Bei der Schädigung durch UV-Strahlung entsteht neben der direkten Schadwirkung ebenfalls hyperosmolarer Stress in der Haut. Die Auswirkungen dieses hyperosmolaren Stresses kann durch die erfindungsgemäß verwendeten Substanzen verringert bzw. verhindert werden.

Weiterhin betrifft die Erfindung die Verwendung von Ethylglycinat zum Schutz der Haut vor der Apoptose (dem programmierten Zelltod). Es wird allgemein diskutiert, dass die Apoptose mit (auch vorzeitigen) Alterungsprozessen insbesondere der Haut in Zusammenhang steht.

Besonders bevorzugt wird die Haut vor der durch osmotischen Stress hervorgerufenen Apoptose geschützt. Es wurde gefunden, dass die erfindungsgemäß verwendeten Substanzen in der Lage sind, die Apoptoserate, welche durch osmolaren, insbesondere hyperosmolaren Stress induziert wird, zu verringern (vgl. Beispiele). Dieser hyperosmolare Stress kann beispielsweise auch durch die Einwirkung von UV-Strahlung auf die Haut ausgelöst werden.

Nach einer weiteren bevorzugten Ausführungsform ist in den erfindungsgemäß zu verwendenden Zubereitungen zwischen 0,005 bis 15 Gew.-% Ethylglycinat bezogen auf die gesamte Zubereitung enthalten.

Gemäß einer besonders bevorzugten Ausführungsform liegt Ethulglycinat in einer kosmetischen oder pharmazeutischen Zubereitung vor.

Ethylglycinat kann sowohl in kosmetischen als auch pharmazeutischen Formulierungen eingesetzt werden.

Nach einer besonders bevorzugten Ausführungsform sind die kosmetischen Formulierungen ausgewählt aus Mund-, Zahn-, Haar- und/oder Haut- bzw. Körperpflegeprodukten.

Bevorzugt eignen sich Formulierungen, die Liposomen enthalten können, da Liposomen in der Lage sind, die erfindungsgemäß zu verwendenden Substanzen besonders gut in tiefere Hautschichten zu transportieren und sie so in die Nähe ihres Wirkortes zu bringen. Liposomen können sowohl in ihrem Inneren wässrige Lösungen und Dispersionen von Substanzen transportieren als auch entsprechende amphiphile Stoffe direkt in der Lipidschicht der Liposomen einlagern, beispielsweise geeignet sind dafür die erfindungsgemäß zu verwendenden Tocopherylester.

Besonders bevorzugt erfolgt die Herstellung geeigneter Liposomen wie in der DE 197 40 092 beschrieben, auf die hiermit in vollem Umfang Bezug genommen wird.

Bevorzugt sind die Formulierungen ausgewählt aus Hautpflegeprodukten, wie beispielsweise wässrige oder alkoholische Lösungen sowie deren Gemische, Gele, Lotionen, Öle oder Cremes. Geeignet ist der Einsatz auch in Sonnencremes, - ölen, -gelen oder -sprays, sowie insbesondere in After-sun-Produkten, da bei durch die UV-Strahlung gereizter, geröteter oder verbrannter Haut, der transepidermale Wasserverlust besonders stark ansteigt. Des weiteren sind die erfindungsgemäßen Substanzen bevorzugt für trockene, rissige, raue, empfindliche oder irritierte Haut einzusetzen, da auch diese über eine z.T. geschädigte Hornschicht überproportional viel Wasser aus den unteren Epidermisschichten verlieren kann.

Ebenfalls geeignet ist die Verwendung in tensidhaltigen Produkten wie Seifen, Duschbädern, Badezusätzen und/oder Shampoos. Verwendet werden können die Substanzen weiterhin in Hautreinigungsprodukten insbesondere für die Gesichtshaut, wie z.B. Gesichtswasser oder Reinigungsmilch. Gesichtspackungen zur Erfrischung und Pflege der Gesichtshaut sind ebenfalls geeignet.

In Haarbehandlungsmitteln, wie z.B. Kuren oder Spülungen zur Haarnachbehandlung, als auch in Haarfärbe- und Dauerwellmitteln, können die Substanzen zum Schutz von Haut und Hautanhangsgebilden, wie den Haaren, und insbesondere den biologisch noch aktiven Teilen des Haares, wie den Haarfollikeln, ebenfalls erfindungsgemäß eingesetzt werden.

Die erfindungsgemäß verwendeten Körperpflegeprodukte können insbesondere auch Desodorantien wie beispielsweise Deosprays, Deostifte oder Deoroller, und/oder Antitranspirationen (Antihidrotika) umfassen, die auf die Haut, insbesondere der Achselhöhle aufgetragen werden, um Geruchsbildung durch Schweißzersetzung bzw. das Schwitzen im allgemeinen zu verhindern. Insbesondere durch das Schwitzen entsteht eine hohe Salzkonzentration auf der Haut, die zu hyperosmolare Bedingungen auf der Haut und somit zu osmotischem Stress führen können.

Weiterhin bevorzugt ist die Verwendung zur Verhinderung von osmolarem Stress in Mund- und/oder Zahnpflegeprodukten, wie beispielsweise Zahncremes, Zahngele, Mundwässer, Mundspülungen, Mundsprays oder Gebiß- und Prothesepflegeprodukten.

Die erfindungsgemäß zu verwendenden Zubereitungen, Mund-, Zahn-, Körper-und Haarpflegemittel sowie Haarfärbemittel, wie beispielsweise Deodorants, Zahncremes, Zahngele, Mundwässer, Mundspülungen, Haarshampoos, Haarlotionen, Schaumbäder, Duschbäder, Cremes, Gele, Lotionen, alkoholische und wässrig/alkoholische Lösungen, Emulsionen, Wachs/ Fett-Massen, Stiftpräparate, Puder oder Salben können - je nach Art der Formulierung - als Hilfs- und Zusatzstoffe milde Tenside, Ölkörper, Emulgatoren, Überfettungsmittel, Perlglanzwachse, Konsistenzgeber, Verdickungsmittel, Polymere, Siliconverbindungen, Fette, Wachse, Stabilisatoren, biogene Wirkstoffe, Deodorantien, Antitranspirantien, Antischuppenmittel, Filmbildner, Quellmittel, UV-Lichtschutzfaktoren, Antioxidantien, Hydrotrope, Konservierungsmittel, Insektenrepellentien, Selbstbräuner, Solubilisatoren, Parfümöle, Farbstoffe und dergleichen enthalten.

Typische Beispiele für geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, $\alpha$-Olefinsulfonate, Ethercarbonsäuren, Alkyloligoglucoside, Fettsäureglucamide, Alkylamidobetaine und/oder Proteinfettsäurekondensate, letztere vorzugsweise auf Basis von Weizenproteinen.

Als Ölkörper kommen beispielsweise Guerbetalkohole auf Basis von Fettalkoholen mit 6 bis 18, vorzugsweise 8 bis 10 Kohlenstoffatomen, Ester von linearen $C_6$-$C_{22}$-Fettsäuren mit linearen $C_6$-$C_{22}$-Fettalkoholen, Ester von verzweigten $C_6$-$C_{13}$-Carbonsäuren mit linearen $C_6$-$C_{22}$-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat; Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmität, Erucylstearat, Erucylisostearat, Erucyloleat, Erucylbehenat und Erucylerucat in Betracht.

Daneben eignen sich Ester von linearen $C_6$-$C_{22}$-Fettsäuren mit verzweigten Alkoholen, insbesondere 2-Ethylhexanol, Ester von Hydroxycarbonsäuren mit linearen oder verzweigten $C_6$-$C_{22}$-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis $C_6$-$C_{10}$-Fettsäuren, flüssige Mono-/Di-/Triglyceridmischungen auf Basis von $C_6$-$C_{18}$-Fettsäuren, Ester von $C_6$-$C_{22}$-Fettalkoholen und/oder Guerbetalkoholen mit aromatischen Carbonsäuren, insbesondere Benzoesäure, Ester von $C_2$-$C_{12}$-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte $C_6$-$C_{22}$-Fettalkoholcarbonate, Guerbetcarbonate, Ester der Benzoesäure mit linearen und/oder verzweigten $C_6$-$C_{22}$-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestern mit Polyolen, Siliconöle und/oder aliphatische bzw. naphthenische Kohlenwasserstoffe, wie z. B. Squalan, Squalen oder Dialkylcyclohexane.

Als Emulgatoren kommen beispielsweise nichtionogene Tenside aus mindestens einer der folgenden Gruppen in Frage:

(1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;

(2) $C_{12}/_{18}$-Fettsäuremono- und -diester von Anlagerungsprodukten von 1 bis 30 Mol Ethylenoxid an Glycerin;

(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;

(4) Alkyl- und/oder Alkenylmono- und -oligoglycoside mit 8 bis 22 Kohlenstoffatomen im Alk(en)ylrest und deren ethoxylierte Analoga;

(5) Anlagerungsprodukte von 15 bis 60 Mol Ethylenoxid an Ricinusöl und/oder gehärtetes Rizinusöl;

(6) Polyol- und insbesondere Polyglycerinester;

(7) Anlagerungsprodukte von 2 bis 15 Mol Ethylenoxid an Ricinusöl und/oder gehärtetes Rizinusöl;

(8) Partialester auf Basis linearer, verzweigter, ungesättigter bzw. gesättigter $C_{6/22}$-Fettsäuren, Rizinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);

(9) Mono-, Di- und Trialkylphosphate sowie Mono-, Di- und/oder Tri-PEGalkylphosphate und deren Salze;

(10) Wollwachsalkohole;

(11) Polysiloxan-Polyalkyl-Polyether-Copolymere bzw. entsprechende Derivate;

(12) Mischester aus Pentaerythrit, Fettsäuren, Citronensäure und Fettalkohol gemäß DE 1165574 PS und/oder Mischester von Fettsäuren mit 6 bis 22 Kohlenstoffatomen, Methylglucose und Polyolen, vorzugsweise Glycerin oder Polyglycerin,

(13) Polyalkylenglycole sowie

(14) Glycerincarbonat.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Rizinusöl stellen bekannte, im Handel erhältliche Produkte dar.

Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. $C_{12/18}$-Fettsäuremono- und -diester von Anlagerungsprodukten von Ethylenoxid an Glycerin sind aus DE 2024051 PS als Rückfettungsmittel für kosmetische Zubereitungen bekannt.

Alkyl- und/oder Alkenylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403), Polyglyceryl Dimerate Isostearate sowie deren Gemische.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer $C_{8/18}$-Alkyl-oder -Acylgruppe im Molekül mindestens eine freie Aminogruppe und mindestens eine -COOH- oder -SO$_3$H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das $C_{12/18}$-Acylsarcosin. Neben den ampholytischen kommen auch quartäre Emulgatoren in Betracht, wobei solche vom Typ der Esterquats, vorzugsweise methylquaternierte Difettsäuretriethanolaminester-Salze, besonders bevorzugt sind.

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron- und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher . Kettenlänge und/oder Polyglycerinpoly-12-hydroxystearaten.

Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium hydroxypropyl hydrolyzed collagen (Lamequat®L/Grünau), quaternierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der FR 2252840 A sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/ . Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/Acrylat-Copolymere, Octylacrylamid/Methylmeth-acrylat/tert.Butylaminoethylmethacrylat/2-Hydroxyproyl-methacrylat-Copolymere, Polyvinylpyrrolidon, Vinylpyrrolidon/Vinylacetat-Copolymere, Vinylpyrrolidon/Dimethylaminoethylmethacrylat/Vinylcaprolactam-Terpolymere sowie gegebenenfalls derivatisierte Celluloseether und Silicone in Frage.

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in Cosm.Toil. 91, 27 (1976).

Typische Beispiele für Fette sind Glyceride, als Wachse kommen u.a. natürliche Wachse, wie z.B. Candelillawachs, Carnaubawachs, Japanwachs, Espartograswachs, Korkwachs, Guarumawachs, Reis-keimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage.

Als Stabilisatoren können Metallsalze von Fettsäuren, wie z.B. Magnesium-, Aluminium- und/oder Zinkstearat bzw. -ricinoleat eingesetzt werden.

Unter biogenen Wirkstoffen sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

Als Antischuppenmittel können Climbazol, Octopirox und Zinkpyrithion eingesetzt werden.

Gebräuchliche Filmbildner sind beispielsweise Chitosan, mikrokristallines Chitosan, quaterniertes Chitosan, Polyvinylpyrrolidon, Vinylpyrrolidon-VinylacetatCopolymerisate, Polymere der Acrylsäurereihe, quaternäre Cellulose-Derivate, Kollagen, Hyaluronsäure bzw. deren Salze und ähnliche Verbindungen.

Als Quellmittel für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in Cosm.Toil. 108, 95 (1993) entnommen werden.

In einer weiteren bevorzugten Ausführungsform enthalten die erfindungsgemäßen kosmetischen Zusammensetzungen mindestens einen organischen oder mineralischen oder modifizierten mineralischen Lichtschutzfilter. Bei den Lichtschutzfiltern handelt es sich um bei Raumtemperatur flüssig oder kristallin vorliegende Substanzen, die in der Lage sind, ultraviolette Strahlen zu absorbieren und die aufgenommene Energie in Form längerwelliger Strahlung, z. B. Wärme wieder abzugeben. Man unterscheidet UVA-Filter und UVB-Filter. Die UVA- und UVB-Filter können sowohl einzeln als auch in Mischungen eingesetzt werden. Der Einsatz von Filter-Mischungen ist erfindungsgemäß bevorzugt.

UVB-Filter können öllöslich oder wasserlöslich sein. Als öllösliche Substanzen sind z.B. zu nennen:

- Derivate von Campher, insbesondere 3-Benzylidencampher bzw. 3-Benzylidennorcampher und dessen Derivate, z.B. 3-(4-Methylbenzy-liden)campher wie in der EP 0693471 B1 beschrieben;
- Dibenzoylmethanderivate;
- Diphenylacrylsäureester und dessen Derivaten;
- Benzimidazolderviate;
- Derivaten von monomeren und oligomeren 4,4-Diarylbutadiencarbonsäureestern und -carbonsäureamiden,
- 2-Aminobenzoesäurederivate
- 4-Aminobenzoesäurederivate, vorzugsweise 4-Dimethylamino)benzoesäure-2-ethylhexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
- Ester der Salicylsäure, vorzugsweise Salicylsäure-2-ethylhexylester, Salicylsäure-4-isopropylbenzylester, Salicylsäurehomomenthylester;
- Derivate des Benzophenons, vorzugsweise 2-Hydroxy-4-methoxybenzo-phenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, vorzugsweise symmetrisch und unsymmetrisch substituierte 1,3,5 Triazine, wie z.B. 2,4,6-Trianilino-(p-carbo-2'-ethyl-1'-hexyloxy)-1,3,5-triazin und Octyl Triazon, wie in der EP 0818450 A1 beschrieben oder Dioctyl Butamido Triazone (Uvasorb® HEB);
- Propan-1,3-dione, wie z.B. 1-(4-tert.Butylphenyl)-3-4'methoxyphenyl)propan-1,3-dion;
- Ketotricyclo(5.2.1.0)decan-Derivate, wie in der EP 0694521 B1 beschrieben. Erfindungsgemäß besonders bevorzugte öllösliche UV-Filter sind 1-(4-tert.-Butylphenyl)-3-(4'-methoxyphenyl)propan-1,3-dion (Parsol® 1789), 1-Phenyl-3-(4'-isopropylphenyl)-propan-1,3-dion, 3-(4'-Methylbenzyliden)-D,L-campher, 4-(Dimethylamino)-benzoesäure-2-ethylhexylester, 4-(Dimethylamino)benzoesäure-2-octylester, 4-(Dimethylamino)-benzoesäureamylester, 4-Methoxyzimtsäure-2-ethylhexylester, 4-Methoxyzimtsäurepropytester, 4-Methoxyzimtsäureisopentylester, 2-Cyano-3,3-phenylzimtsäure-2-ethylhexylester (Octocrylene), Salicylsäure-2-ethylhexylester, Salicylsäure-4-isopropylbenzylester, Salicylsäurehomomenthylester (3,3,5-Trimethyl-cyclohexylsalicylat), 2-Hydroxy-4-methoxybenzophenon, 2-Hydroxy-4-methoxy-4'-methylbenzophenon, 2,2'-Dihydroxy-4-methoxybenzophenon, 4-Methoxybenzmalonsäuredi-2-ethylhexyf-ester, 2,4,6-Trianilino-(p-carbo-2'-ethyl-1'-hexyloxy)-1,3,5-triazin (Octyl Triazone) und Dioctyl Butamido Triazone (Uvasorb® HEB) sowie beliebige Mischungen der genannten Komponenten.

Als wasserlösliche Substanzen kommen in Frage:

- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)-sulfonsäure und deren Salze.

Als typische UV-A-Filter kommen insbesondere Derivate des Benzoylmethans in • Frage, wie beispielsweise 1-(4'-tert.Butylpheny))-3-(4'-methoxypheny))propan-1,3-dion, 4-tert.-Butyl-4'-methoxydibenzoylmethan (Parsol 1789), 1-Phenyl-3-(4'-isopropylphenyl)-propan-1,3-dion sowie Enaminverbindungen, wie beschrieben in der DE 19712033 A1 (BASF). Die UV-A und UV-B-Filter können selbstverständlich auch in Mischungen eingesetzt werden.

Neben den genannten löslichen Stoffen kommen für diesen Zweck auch unlösliche Lichtschutzpigmente, nämlich feindisperse Metalloxide bzw. Salze in frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in SÖFW-Journal 122, 543 (1996) zu entnehmen.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butylund Lauryl-, Palmitoyl-, Oleyl-, $\gamma$-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis $\mu$mol/kg), ferner (Metall)-Chelatoren (z.B. $\alpha$-Hydroxyfettsäuren, Palmitinsäure, Phytinsäure, Lactoferrin), $\alpha$-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. $\gamma$-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, $\alpha$-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, $ZnSO_4$) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind

- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

Als Konservierungsmittel eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen. Als Insekten-Repellentien kommen N,N-Diethyl-m-toluamid, 1,2-Pentandiol oder Ethyl Butylacetylaminopropionate in Frage, als Selbstbräuner eignet sich Dihydroxyaceton.

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, $\propto$-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, Verlag Chemie, Weinheim, 1984, S.81-106 zusammengestellt sind. Diese Farbstoffe werden üblicherweise in Konzentrationen von 0,001 bis 0,1 Gew.-%, bezogen auf die gesamte Mischung, eingesetzt.

Die erfindungsgemäß zu verwendenden Zubereitungen enthalten weiterhin Feuchthaltemittel zum Schutz vor Austrocknung sowie zur Konsistenzregelung und Kältestabilität der Produkte. Sie können aber ferner auch zur Suspensionsvermittlung und zur Geschmacks- oder Glanzbeeinflussung dienen.

Gewöhnlich werden als Feuchthaltemittel toxikologisch unbedenkliche Polyole, wie beispielsweise Sorbitol, Xylitol, Glycerin, Mannitol, 1,2-Propylenglycol oder Gemische davon verwendet, aber auch Polyethylenglycole mit Molekulargewichten von 200 - 2000, insbesondere von 200 bis 1500, können als Feuchthaltemittelkomponenten in Zahncremes dienen.

Bevorzugt ist die Kombination mehrerer Feuchthaltemittelkomponenten, wobei die Kombination von Glycerin und/oder Sorbitol mit einem Gehalt an 1,2-Propylenglycol oder Polyethylenglycol als besonders bevorzugt anzusehen ist.

Auch die Kombination von Xylitol mit einem oder beiden der Feuchthaltemittel Glycerin und Sorbitol ist als bevorzugte Kombination zu nennen, wobei neben der Eigenschaft als Feuchthaltemittel auch die antikariogene Eigenschaft des Xylitols zum nennen ist.

Je nach Produkttyp, insbesondere bei Mund- und/oder Zahnpflegemitteln ist das Feuchthaltemittel oder das Gemisch aus Feuchthaltemitteln in der Gesamtzusammensetzung in einer Menge von 10 - 85 Gew.-%, vorzugsweise 20 - 70 Gew.-% und insbesondere 30 - 50 Gew.-% enthalten.

Weiterhin können insbesondere die erfindungsgemäß verwendeten Mund-und/oder Zahnpflegemitteln noch Antikaries-Wirkstoffe, Antizahnstein-Wirkstoffe, remineralisierende und/oder antimikrobielle Wirkstoffe sowie Geschmacksstoffe, Putzkörper oder Poliermittel enthalten.

Zur Bekämpfung von und Vorbeugung gegen Karies eignen sich vor allem Fluorverbindungen, bevorzugt aus der Gruppe der Fluoride oder Monofluorophosphate in einer Menge von 0,1 - 0,5 Gew.-% Fluor. Geeignete Fluorverbindungen sind z. B. Natriumfluorid, Kaliumfluorid, Zinnfluorid, Dinatriummonofluorophosphat ($Na_2PO_3F$), Dikaliummonofluorophosphat oder das Fluorid einer organischen Aminoverbindung. Organische Aminfluoride im Sinne der Erfindung sind beispielsweise Ammoniumfluorid, Cetylaminhydrofluorid und Bis-(hydroxyethyl)-aminopropyl-N-hydroxyethyloctadecylamindihydrofluorid.

In einer besonders bevorzugten Ausführungsform der Erfindung wird dem Mund-und Zahnpflegemittel- Natriumfluorid, Ammoniumfluorid oder ein organisches Aminfluorid zugesetzt.

Als antimikrobielle Komponenten eignen sich z. B. Phenole, Resorcine, Bisphenole, Salicylanilide und -amide sowie deren halogenierte Derivate, halogenierte Carbanilide und p-Hydroxybenzoesäureester.

Unter den antimikrobiellen Komponenten sind diejenigen besonders geeignet, die das Wachstum von Plaque-Bakterien hemmen. Beispielsweise sind halogenierte Diphenylether, wie 2,4-Dichlor-2'-hydroxydiphenylether, 4,4'-Dichlor-2'-hydroxydiphenylether, 2,4,4'-Tribrom-2'-hydroxydiphenylether, 2,4,4'-Trichlor-2'-hydroxydiphenylether (Triclosan) als antimikrobielle Wirkstoffe geeignet. Neben Bromchlorophen, Phenylsalicylsäureestern und 5-Amino-1,3-bis(2-ethylhexyl)-hexahydro-5-methylpyrimidin (Hexetidin) wirken auch Zink- und Kupferionen antimikrobiell, wobei synergistische Effekte insbesondere in Kombination mit Hexetidin und Triclosan auftreten. Auch quartäre Ammoniumverbindungen, wie zum Beispiel Cetylpyridiniumchlorid, Benzalkoniumchlorid, Domiphenbromid und Dequaliniumchlorid sind einsetzbar. Als antimikrobiell wirksam haben sich auch Octapinol, Octenidine und Sanguinarin erwiesen. Weiterhin bevorzugt geeignet sind auch Bisbiduanid-Derivate bzw. deren Salze, insbesondere Chlorhexidin und Alexidin, Die antimikrobiellen Wirkstoffe werden bevorzugt in Mengen von 0,01 - 1 Gew.-% in den erfindungsgemäßen Mitteln eingesetzt. Besonders bevorzugt wird Irgacare® MP in einer Menge von 0,01 - 0,3 Gew.-% verwendet.

Bei Zahnstein handelt es sich um Mineralablagerungen, die dem natürlichen Zahnschmelz sehr ähnlich sind. Um eine Zahnsteinbildung zu inhibieren, werden den erfindungsgemäßen Zahnreinigungsmitteln Stoffe zugesetzt, die gezielt in die Kristallkeimbildung eingreifen und bereits vorhandene Keime am Weiterwachsen hindern. Hierbei handelt es sich beispielsweise um kondensierte Phosphate, die bevorzugt gewählt werden aus der Gruppe der Tripolyphosphate, der Pyrophophate, der Trimetaphosphate oder deren Gemischen. Sie werden in Form ihrer Alkali- oder Ammoniumsalze, bevorzugt in Form ihrer Natrium- oder Kaliumsalze eingesetzt. Wässrige Lösungen dieser Phosphate reagieren typischerweise alkalisch, so dass der pH-Wert der erfindungsgemäßen Zahnpflegemittel ggf. durch Zusatz von Säure auf Werte von 7,5 - 9 eingestellt wird. Als Säuren können dabei z. B. Zitronensäure, Phosphorsäure oder saure Salze, z. B. $NaH_2PO_4$ verwendet werden. Der gewünschte pH-Wert des Zahnpflegemittels kann aber auch durch Zusatz saurer Salze der kondensierten Phosphate, also z. B. $K_2H_2P_2O_7$, eingestellt werden.

Auch Gemische verschiedener kondensierter Phosphate und/oder hydratisierte Salze der kondensierten Phosphate sind erfindungsgemäß einsetzbar. Zahnsteininhibitoren werden üblicherweise in Mengen von 0,1 - 5 Gew.-%, bevorzugt 0,1 - 3 Gew.-% und insbesondere 0,1 - 2 Gew.-% in den erfindungsgemäßen Mitteln eingesetzt. Weitere geeignete Zahnsteininhibitoren sind Organophosphonate wie 1-Hydroxyethan-1,1-diphosphonat (Na-Salz) und Zinkcitrat.

Die erfindungsgemäßen Mittel enthalten vorzugsweise auch Stoffe, die eine Remineralisierung des Zahnschmelzes fördern und Dentalläsionen zu schließen vermögen. Diese sind üblicherweise in einer Gesamtmenge von 0,1 - 10 Gew.-%, vorzugsweise 0,1 - 5 Gew.-% und insbesondere 0,1 - 3 Gew.-% enthalten. Hierzu gehören z. B. Fluoride, Phosphatsalze des Calciums wie z. B. Calciumglycerinphosphate, Calciumhydrogenphopsphat, Hydroxylapatit, Fluorapatit, F-dotierter Hydroxylapatit, Dicalciumphosphatdihydrat sowie Calciumfluorid. Aber auch Magnesiumsalze wie z. B. Magnesiumsulfat, Magnesiumfluorid oder Magnesiummonofluorophosphat wirken remineralisierend.

Für den Fall, dass die erfindungsgemäß verwendeten Mund- und Zahnpflegemittel als Mundwasser konfektioniert werden, können sie weiterhin Ethanol in Einsatzmengen von 0 bis 10 Gew.-%, bevorzugt in Mengen von 1 bis 5 Gew.-%, bezogen auf das Gesamtgewicht des Mittels, enthalten.

Vorzugsweise enthalten die erfindungsgemäß zu verwendenden Mittel Geschmacksstoffe, zu denen z. B. Süßungsmittel und/oder Aromaöle gehören. Als Süßungsmittel eignen sich beispielsweise Saccharinate (insbesondere Natriumsaccharinat), Cyclamate (insbesondere Natriumcyclamat) sowie Sucrose, Lactose, Maltose oder Fructose. Als Aromaöle kommen alle für Mund- und Zahnpflegemittel gebräuchlichen natürlichen und synthetischen Aromen in Frage. Natürliche Aromen können sowohl in Form der aus den Drogen isolierten etherischen Öle (Mischung) als auch in Form der hieraus isolierten Einzelkomponenten verwendet werden. Bevorzugt sollte wenigstens ein Aromaöl aus der Gruppe Pfefferminzöl, Krausenminzöl, Anisöl, Sternanisöl, Kümmelöl, Eukalyptusöl, Fenchelöl, Zimtöl, Nelkenöl, Geraniumöl, Salbeiöl, Pimentöl, Thymianöl, Majoranöl, Basilikumöl, Citrusöl, Gaultheriaöl oder eine/mehrere daraus isolierte bzw. synthetisch erzeugte Komponenten dieser Öle enthalten sein. Die wichtigsten Komponenten der genannten Öle sind z. B. Menthol, Carvon, Anethol, Cineol, Eugenol, Zimtaldehyd, Caryophyllen, Geraniol, Citronellol, Linalool, Salven, Thymol, Terpinen, Terpinol, Methylchavicol und Methylsalicylat. Weitere geeignete Aromen sind z. B. Menthylacetat, Vanillin, Jonone, Linalylacetat, Rhodinol und Piperiton.

Für den Fall, dass die erfindungsgemäß verwendeten Mund- und Zahnpflegemittel als Zahnpasten, Zahngele, oder flüssige transparente Zahnputzmittel konfektioniert werden, enthalten sie als weiteren Bestandteil mindestens einen Putzkörper.

Die Putzkörper dienen zur mechanischen Entfernung des unverkalkten Zahnbelags und sollen idealerweise zur Glanzgebung der Zahnoberfläche (Poliereffekt) bei gleichzeitiger minimaler Scheuerwirkung (Abrasionseffekt) und Schädigung des Zahnschmelzes und des Dentins führen. Das Abrasionsverhalten der Putzkörper wird im Wesentlichen durch deren Härte, Korngrößenverteilung und Oberflächenstruktur bestimmt. Bei der Auswahl geeigneter Putzkörper werden folglich insbesondere solche bevorzugt ausgewählt, die bei hoher Reinigungsleistung über eine minimale Abrasionswirkung verfügen. Heutzutage werden als Putzkörper vorwiegend Stoffe verwendet, die kleine Korngrößen aufweisen und weitgehend frei von scharfen Ecken und Kanten sind.

Üblicherweise werden als Putzkörper oder Poliermittel wasserlösliche anorganische Stoffe eingesetzt. Besonders vorteilhaft ist die Verwendung sehr feinteiliger Poliermittel mit einer mittleren Korngröße von 1 - 200 $\mu$m, vorzugs-

weise 1 - 50 μm und insbesondere 1-10 μm.

Prinzipiell können die erfindungsgemäßen Poliermittel ausgewählt sein aus Kieselsäuren, Aluminiumhydroxid, Aluminiumoxid, Silikaten, organischen Polymeren oder Gemischen davon. Weiterhin können aber auch sogenannte Metaphosphate sowie calciumhaltige Polierkomponenten in Mengen bis zu 5 Gew.-% in den erfindungsgemäßen Mitteln enthalten sein.

Es kann erfindungsgemäß bevorzugt sein, Kieselsäuren als Poliermittel in Zahnpasten oder flüssigen Zahnreinigungsmitteln einzusetzen. Man unterscheidet unter den Kieselsäure-Poliermitteln grundsätzlich zwischen Gelkieselsäuren, Hydrogelkieselsäuren und Fällungskieselsäuren. Fällungs- und Gelkieselsäuren sind erfindungsgemäß besonders bevorzugt, da sie bei ihrer Herstellung breit variiert werden können und besonders gut mit Fluorid-Wirkstoffen verträglich sind. Sie eignen sich weiterhin auch besonders gut für die Herstellung von Gel- oder Liquid-Zahncremes.

Gelkieselsäuren werden durch die Umsetzung von Natriumsilikatlösungen mit starken, wässrigen Mineralsäuren unter Ausbildung eines Hydrosols, Alterung zum Hydrogel, Waschen und anschließendem Trocknen erzeugt. Erfolgt die Trocknung unter schonenden Bedingungen auf Wassergehalte von 15 bis 35 Gew.-%, so werden sogenannte Hydrogelkieselsäuren erhalten, wie sie beispielsweise auch in der US 4,153,680 beschrieben sind. Durch Trocknung dieser Hydrogelkieselsäuren auf Wassergehalte unterhalb von 15 Gew.-% erfolgt eine irreversible Schrumpfung der vorher lockeren Struktur zur dichten Struktur des sogenannten Xerogels. Solche Xerogelkieselsäuren sind beispielsweise aus der US 3,538,230 bekannt.

Eine zweite, bevorzugt geeignete Gruppe von Kieselsäure-Poliermitteln sind die Fällungskieselsäuren. Diese werden durch Ausfällung von Kieselsäure aus verdünnten Alkalisilikat-Lösungen durch Zugabe von starken Säuren unter Bedingungen erhalten, bei denen die Aggregation zum Sol und Gel nicht eintreten kann. Geeignete Verfahren zur Herstellung von Fällungskieselsäuren sind beispielsweise in der DE-OS 25 22 586 und in der DE-OS 31 14 493 beschrieben. Erfindungsgemäß besonders geeignet ist eine gemäß der DE-OS 31 14 493 hergestellte Fällungskieselsäure mit einer BET-Oberfläche von 15 - 110 m$^2$/g, einer Partikelgröße von 0,5 bis 20 μm, wobei wenigstens 80 Gew.-% der Primärpartikel unter 5 μm liegen sollen, und einer Viskosität in 30%iger Glycerin-Wasser-(1:1)-Dispersion von 30 - 60 Pa s (20°C) in einer Menge von 10 - 20 Gew.-% der Zahnpaste. Bevorzugt geeignete Fällungskieselsäuren dieser Art weisen außerdem gerundete Ecken und Kanten auf und sind unter der Handelsbezeichnung Sident®12 DS der Firma Degussa erhältlich.

Weitere Fällungskieselsäuren dieser Art sind Sident®8 der Firma Degussa und Sorbosil®AC 39 der Firma Crosfield Chemicals. Diese Kieselsäuren zeichnen sich durch eine geringere Verdickungswirkung und eine etwas höhere mittlere Teilchengröße von 8 - 14 μm bei einer spezifischen Oberfläche von 40 - 75 m$^2$/g (nach BET) aus und eignen sich besonders gut für flüssige Zahncremes. Diese sollten eine Viskosität (25°C, Scherrate D = 10 s$^{-1}$) von 10 - 100 Pa s aufweisen.

Des weiteren können die Kieselsäuren vom Typ Zeodent® der Firma Huber-Corp., Tixosil® der Firma Rhodia sowie weitere Sorbosil-Typen in den erfindungsgemäßen Mitteln eingesetzt werden. Besonders bevorzugt sind Zeodent®113, Tixosil® 123 und 73 sowie Sorbosil®AC33.

Zahnpasten, die eine deutlich höhere Viskosität von mehr als 100 Pa s (25°C, D = 10 s$^{-1}$) aufweisen, benötigen hingegen einen genügend hohen Anteil an Kieselsäuren mit einer Teilchengröße von weniger als 5 μm, bevorzugt wenigstens 3 Gew.-% einer Kieselsäure mit einer Partikelgröße von 1 bis 3 μm. Solchen Zahnpasten setzt man daher bevorzugt neben den genannten Fällungskieselsäuren noch feinteiligere, sogenannte Verdickungskieselsäuren mit einer BET-Oberfläche von 150 - 250 m$^2$/g zu. Als Beispiele für Handelsprodukte, die die genannten Bedingungen erfüllen, sind insbesondere Sipernat®22 LS oder Sipernat®320 DS der Firma Degussa zu nennen.

Als Aluminiumoxid-Poliermittel eignet sich bevorzugt eine schwach calcinierte Tonerde mit einem Gehalt von wenigstens 10 Gew.-%, von Alpha-Aluminiumoxid verschiedener, sogenannter Gamma-Aluminiumoxid-Modifikationen.

Geeignete schwach calcinierte Tonerden werden durch Calcination aus Aluminiumhydroxid hergestellt. Aluminiumhydroxid geht durch Calcination in das bei Temperaturen oberhalb 1200°C thermodynamisch stabile α-Al$_2$O$_3$ über. Die bei Temperaturen zwischen 400 und 1000°C auftretenden, thermodynamisch instabilen Al$_2$O$_3$-Modifikationen bezeichnet man als Gamma-Formen (vgl. Ullmann, Enzyklopädie der technischen Chemie, 4. Auflage (1974), Band 7, Seite 298). Durch Wahl der Temperatur und der Zeitdauer bei der Calcination kann man den Calcinationsgrad, d.h. die Umwandlung in das thermodynamisch stabile α-Al$_2$O$_3$ auf beliebige Höhe einstellen. Man erhält durch schwache Calcination eine Tonerde mit einem Gehalt an γ-Al$_2$O$_3$, der um so niedriger ist, je höher die Calcinationstemperatur und je länger die Calcinationsdauer gewählt wird. Schwach calcinierte Tonerden unterscheiden sich von reinem α-Al$_2$O$_3$ durch eine geringere Härte der Agglomerate, eine größere spezifische Oberfläche und größere Porenvolumina.

Der Dentinabrieb (RDA) der erfindungsgemäß zu verwendenden schwächer calcinierten Tonerden mit einem Anteil von 10 - 50 Gew.-% γ-Al$_2$O$_3$ beträgt nur 30- 60 % des Dentinabriebs eines stark calcinierten, reinen α-Al$_2$O$_3$ (gemessen in einer Standard-Zahnpaste mit 20 Gew.-% Tonerde als einzigem Poliermittel).

Im Gegensatz zu α-Al$_2$O$_3$ läßt sich das γ-Al$_2$O$_3$ mit einer wässrigammoniakalischen Lösung von Alizarin S (1,2-Dihydroxy-9,10-anthrachinon-4= sulfonsäure) rot anfärben. Man kann den Grad der Anfärbbarkeit als Maß für den Calcinationsgrad bzw. für den Anteil an δ-Al$_2$O$_3$ in einer calcinierten Tonerde wählen :

Ca. 1 g $Al_2O_3$, 10 ml einer Lösung von 2 g/l Alzarin S in Wasser und 3 Tropfen einer wässrigen, 10 Gew.-%igen Lösung von NH3 werden in ein Reagenzglas gegeben und kurz aufgekocht. Das $Al_2O_3$ wird anschließend abfiltriert, nachgewaschen, getrocknet und unter dem Mikroskop beurteilt oder farbmetrisch ausgewertet.

Geeignete, schwach calcinierte Tonerden mit einem Gehalt von 10 - 50 Gew.-% $\gamma$-$Al_2O_3$ lassen sich nach diesem Verfahren schwach bis tief rosa anfärben.

Aluminiumoxid-Poliermittel verschiedener Calcinationsgrade, Mahlfeinheit und Schüttgewichte sind im Handel erhältlich, z.B. die "Poliertonerden" der Firma Giulini-Chemie beziehungsweise ALCOA.

Eine bevorzugt geeignete Qualität "Poliertonerde P10 feinst" weist eine Agglomeratgröße unter 20 $\mu$m, eine mittlere Primärkristallgröße von 0,5 - 1,5 $\mu$m und ein Schüttgewicht von 500 - 600 g/l auf.

Die Verwendung von Silikaten als Poliermittelkomponenten kann ebenfalls erfindungsgemäß bevorzugt sein. Sie werden insbesondere in der modernen Praxis als Putzkörper eingesetzt. Beispiele für erfindungsgemäß einsetzbare Silikate sind Aluminiumsilikate und Zirkoniumsilikate. Insbesondere das Natrium-Aluminiumsilikat der empirischen Formel $Na_{12}(AlO_2)_{12}(SiO_2)_{12}$ x $7H_2O$ kann als Poliermittel geeignet sein, wie beispielsweise das synthetische Zeolith A.

Beispiele für erfindungsgemäße wasserunlösliche Metaphosphate sind vor allem Natriummetaphosphat, Calciumphosphat wie beispielsweise Tricalciumphosphat, Calciumhydrogenphosphat, Calciumhydrogenphosphat-Dihydrat und Calciumpyrophosphat.

Des weiteren können erfindungsgemäß Magnesiumcarbonat, Magnesiumhydrogenphosphat, Trimagnesiumphosphat oder Natriumhydrogen-carbonat als Poliermittel, insbesondere als Mischung mit anderen Poliermitteln, eingesetzt werden.

Die erfindungsgemäß zu verwendenden Mund- und Zahnpflegemittel können vorzugsweise eine Reihe weiterer Komponenten enthalten. Hierzu gehören u. a.:

- Vitamine, z. B. Retinol, Biotin, Tocopherol, Ascorbinsäure und deren Derivate (z. B. Ester, Salze);
- Pigmente, z. B. Titandioxid oder Zinkoxid;
- Gefärbte Pigmentpartikel, beispielsweise gefärbte Kieselsäurepartikel, wie sie z. B. unter der Verkaufsbezeichnung Sorbosil®BFG 51, BFG 52 und BFG 53 oder Sorbosil®2352 im Handel sind. Es können auch Gemische unterschiedlich gefärbter Pigmentpartikel verwendet werden. Solche, z. B. kräftig orange, rot oder blau gefärbten Gelkieselsäure-Partikel können in Mengen von 0,1 - 1,0 Gew.-% in den erfindungsgemäßen Mitteln enthalten sein;
- Bleichmittel;
- Farbstoffe;
- pH-Stellmittel und Puffersubstanzen, z. B. Natriumcitrat, Natriumbicarbonat oder Kalium- und Natriumphosphate,
- Natriumbenzoat;
- wundheilende und entzündungshemmende Stoffe wie z. B. Allantoin, Harnstoff, Panthenol, Azulen oder Kamillenextrakt, Acetylsalicylsäure-Derivate, Rhodanid; Wasserstoffperoxid;
- Zink- und Mangansulfat.

Bezüglich weiterer fakultativer Komponenten sowie den eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989 oder W. Umbach, Kosmetik: Entwicklung, Herstellung und Anwendung kosmetischer Mittel, 2. Auflage, Thieme Verlag, Stuttgart 1995, verwiesen.

Die Mund- und Zahnpflege im Sinne der Erfindung ist als tägliche kosmetische, nicht-therapeutische Behandlung zu verstehen. Dabei werden die Zähne gereinigt und von Verfärbungen und Essrückständen befreit, was ihr kosmetisches Äußeres schöner und ansehnlicher macht.

Desodorantien bzw. Antitranspiratien - können darüber hinaus die bereits genannten antimikrobiellen Stoffen sowie ätherische Öle oder Parfümöle (insbesondere die bereits genannten) enthalten.

Als Deowirkstoffe kommen weiterhin z.B. Antiperspirantien wie etwa Aluminiumchlorhydrate in Frage. Hierbei handelt es sich um farblose, hygroskopische Kristalle, die an der Luft leicht zerfließen und beim Eindampfen wäßriger Aluminiumchloridlösungen anfallen. Aluminiumchlorhydrat wird zur Herstellung von schweißhemmenden und desodorierenden Zubereitungen eingesetzt und wirkt wahrscheinlich über den partiellen Verschluß der Schweißdrüsen durch Eiweiß- und/oder Polysaccharidfällung [vgl. J.Soc.Cosm.Chem. 24, 281 (1973)]. Unter der Marke Locron® der Hoechst AG, Frankfurt/FRG, befindet beispielsweise sich ein Aluminiumchlorhydrat im Handel, das der Formel $[Al_2(OH)_5Cl]*2,5$ $H_2O$ entspricht und dessen Einsatz besonders bevorzugt ist [vgl. J.Pharm.Pharmacol. 26, 531 (1975)]. Neben den Chlorhydraten können auch Aluminiumhydroxylactate sowie saure Aluminium/Zirko-niumsalze eingesetzt werden. Als weitere Deowirkstoffe können Esteraseinhibitoren zugesetzt werden. Hierbei handelt es sich vorzugsweise um Trialkylcitrate wie Trimethylcitrat, Tripropylcitrat, Triisopro-pylcitrat, Tributylcitrat und insbesondere Triethylcitrat (Hydagen® CAT, Henkel KGaA, Düssel-dorf/FRG). Die Stoffe inhibieren die Enzymaktivität und reduzieren dadurch die Geruchsbildung. Wahrscheinlich wird dabei durch die Spaltung des Citronensäureesters die freie Säure freigesetzt, die den pH-Wert auf der Haut soweit absenkt, daß dadurch die Enzyme inhibiert werden. Weitere Stoffe, die als Esteraseinhibitoren in Betracht kommen, sind Dicarbonsäuren und deren Ester, wie beispielsweise Glutarsäure, Glutarsäuremonoethylester, Glutarsäurediethylester, Adipinsäure, Adipinsäuremono-ethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester. Antibakterielle Wirkstoffe, die die Keimflora beeinflussen und schweißzersetzende Bakterien abtöten bzw. in ihrem Wachstum hemmen, können ebenfalls in den Stiftzubereitungen enthalten sein. Beispiele hierfür sind Chitosan, Phenoxyethanol und Chlorhexidingluconat. Besonders wirkungsvoll hat sich auch 5-Chlor-2-(2,4-dichlorphen-oxy)-phenol erwiesen, das unter der Marke Irgasan® von der Ciba-Geigy, Basel/CH vertrieben wird.

Der Gesamtanteil der Hilfs- und Zusatzstoffe kann 1 bis 50, vorzugsweise 5 bis 40 Gew.-% - bezogen auf die Mittel - betragen. Die Herstellung der Kosmetika, Mund-, Zahn- und Körperpflegemittel kann durch übliche Kalt - oder Heißprozesse erfolgen; vorzugsweise arbeitet man nach der Phaseninversionstemperatur-Methode.

Gemäß eine weiteren besonders bevorzugten Ausführungsform wird die Substanz in pharmazeutischen Zubereitungen eingesetzt.

Je nach Art der Formulierung können die erfindungsgemäßen pharmazeutischen Zubereitungen mindestens einen weiteren Hilfs- oder Zusatzstoff, wie z. B. Öle, Schutzkolloide, Weichmacher, Antioxidantien und/oder Emulgatoren enthalten.

Im Falle einer Dispersion, insbesondere im Falle einer Suspension oder Emulsion, ist es vorteilhaft, zusätzlich ein physiologisch verträgliches Öl wie beispielsweise Sesamöl, Maiskeimöl, Baumwollsaatöl, Sojabohnenöl oder Erdnußöl, Ester mittelkettiger pflanzlicher Fettsäuren oder Fischöle wie beispielsweise Makrelen-, Sprotten- oder Lachsöl zu verwenden.

Ebenfalls geeignete pharmazeutische Applikationsformen sind Puder oder Salben. Zur Erhöhung der Stabilität des Wirkstoffes gegen oxidativen Abbau ist es vorteilhaft, Stabilisatoren wie α-Tocopherol, t-Butylhydroxy-toluol, t-Butylhydroxyanisol, Ascorbinsäure oder Ethoxyquine zuzusetzen.

Geeignet sind die erfindungsgemäß zu verwendenden Substanzen insbesondere für die dermatologische Anwendung durch topische Applikation. Insbesondere zur Wundheilung und zur Behandlung von atopischer Dermatitis, Psoriasis oder Windelekzem kann die Substanz vorteilhafterweise eingesetzt werden. Durch die osmoprotektive Wirkung der erfindungsgemäß verwendeten Substanzen kann so die gereizten, angegriffenen oder erkrankten Haut, insbesondere vor den Folgen von osmotischem Stress, geschützt werden. Geeignete pharmazeutische Zubereitungen sind unter anderem Puder, Salben, Öle, Cremes, Pasten, Suspensionen.

Nach einer weiteren bevorzugten Ausführungsform wird die Substanz zur Applikation auf feste Träger aufgetragen, insbesondere Windeln, Binden, Slip-oder Inkontinenzeinlagen, Verbände oder Pflaster sowie Tüchern für Reinigung und Pflege der Haut. Dadurch ist es möglich, die erfindungsgemäß zu verwendenden Substanzen direkt auf die betroffenen Stellen aufzubringen und sie häufig auch länger einwirken zu lassen, als es bei der einfachen topischen Applikation der Fall wäre.

Gemäß einer besonders bevorzugten Ausführungsform wird die Substanz in kosmetischen oder pharmazeutischen Formulierungen auf den festen Träger aufgetragen. Vorteilhafterweise kann so die Einwirkung der erfindungsgemäß verwendeten Substanzen durch die geeignete, dem Fachmann bekannte Wahl der Formulierungen verbessert und auf die speziellen Bedürfnisse angepasst werden.

Die folgenden Beispiele sollen die Erfindung erläutern, ohne sie darauf zu beschränken:

Beispiel:

**1. Auswirkungen von osmotischem Stress auf die Proliferation von humanen Keratinocyten**

Der Einfluss von hyperosmotischem Stress wird an der humanen Keratinocytenzelllinie HaCaT (vgl. Boukamp, P., Petrussevska, R. T., Breitkreutz, D., Hornung, J., Markham, A., and Fusenig, N.E. (1988) Normal keratinization in a spontaneously immortalized aneuploid human keratinocyte cell line, J. Cell Biol. 106 (3), 761-771) untersucht.

Die Kultivierung der Zellen erfolgt im Inkubator bei 37°C, 5% (v/v) $CO_2$ und wasserdampfgesättigter Atmosphäre. Das entwickelte zelluläre Testsystem ist die folgt aufgebaut: 40.000 Zellen/$cm^2$ werden in isoosmolarem Kulturmedium (Hanks' Basalmedium (vgl. Tabelle 1), 0,002 M L-Glutamin, 5% (v/v) Fötales Kälberserum; Osmolarität: 0,3 osM) ausgesät und für 24 h inkubiert. Das Medium wird entfernt, der Zellrasen einmal mit phosphatgepufferter Salzlösung (PBS) gewaschen und für 1 bis 48 h mit hyperosmolarem Kulturmedium (Osmolarität: 0,5 osM; vgl. Tabelle 2) inkubiert.

Zur Bestimmung des Einflusses verschiedener Osmolyte werden diese in Konzentrationen von 0,1 M bis 1 x $10^{-8}$ M (Glycin, Taurin, Betain, Diglycin) bzw. 1 x $10^{-3}$ M bis 1 x $10^{-9}$ M (dl-α-Tocopherylmonoglycinat bzw. -prolinat) dem hyperosmolaren Kulturmedium zugesetzt, die Zellen für 8 h kultiviert und dann die Proliferationsrate ermittelt. Für die Glycerin- und Inositolmessungen wurden Konzentrationen von 1,0 x $10^{-2}$ bis 1,0 x $10^{-5}$ M verwendet.

Tabelle 1: Zusammensetzung des HANKS' Basalmediums

| a) Standardformulierung (5 l) | |
|---|---|
| **Komponente:** | **Volumen [ml]** |
| HANKS' Salzlösung (10x) ohne NaHCO$_3$, Fa. Biochrom, Kat. Nr.: L2025 | 400 |
| Milli-Q-Wasser | 3600 |
| MEM-Vitamine (100x) Fa. Biochrom, Kat. Nr.: K0373 | 160 |
| MEM-Aminosäuren (essentielle) Fa. Biochrom, Kat. Nr.: K0363 | 318 |
| Nicht essentielle Aminosäuren (100x) Fa. Biochrom, Kat. Nr.: K0293 | 160 |
| Natriumbicarbonat, 7,5% (w/v) Fa. Biochrom, Kat. Nr.: L1713 | 166 |
| Penicillin/Streptomycin 10 mg/ml Fa. Biochrom, Kat. Nr.: A2213 | 50 |

pH-Wert auf 6,8 - 7,2 einstellen und Volumen mit Milli-Q-Wasser auf 5000 ml ergänzen.

Tabelle 2: Basalmedium für die Kultivierung unter osmolarem Stress (2 l)

| **Komponente:** | **Volumen [ml]:** |
|---|---|
| HANKS' Salzlösung (1x) ohne NaHCO$_3$, Fa. Invitrogen/Gibco, Kat. Nr.: 041-94542 | 1600 |
| Milli-Q-Wasser | 59 |
| MEM-Vitamine (100x) Fa. Biochrom, Kat. Nr.: K0373 | 64 |
| MEM-Aminosäuren (essentielle) Fa. Biochrom, Kat. Nr.: K0363 | 127 |
| Nicht essentielle Aminosäuren (100x) Fa. Biochrom, Kat. Nr.: K0293 | 64 |
| Natriumbicarbonat, 7,5% (w/v) Fa. Biochrom, Kat. Nr.: L1713 | 66 |
| Penicillin/Streptomycin 10 mg/ml Fa. Biochrom, Kat. Nr.: A2213 | 20 |

Ansetzen isoosmolaren Kulturmediums:     8,0 g/l NaCl
Ansetzen hyperosmolaren Kulturmediums:   13,8 g/l NaCl

Der pH-Wert sollte nach Zugabe der Komponenten bei 7,0 liegen.

**2. Proliferationsmessung:**

Die Bestimmung der Zellteilungsrate erfolgt mit Hilfe des käuflichen "Cell Proliferation ELISA-BrdU" der Fa. Roche Diagnostics, Mannheim.

Sämtliche Arbeitsschritte werden, falls nicht gesondert darauf hingewiesen wird, bei Raumtemperatur unter leichtem Schütteln auf dem Kippschüttler (Heidolph, Kelheim, Stufe 4) durchgeführt. Die angegebenen Volumina beziehen sich auf eine Kavität einer 96-Lochplatte. Lösungen werden durch Umdrehen der ELISA-Platte mit anschließendem kräftigen Ausschlagen auf saugfähigem Papier entfernt.

10 $\mu$l der 1:100 BrdU-Lösung (Endkonzentration 10 $\mu$M BrdU) werden dem Kulturmedium zugegeben und die Zellen für 1 h unter Kulturbedingungen inkubiert. Das Kulturmedium wird entfernt, 200 $\mu$l der Fixierlösung aufgetragen und die Ansätze für 16 h bei 4°C inkubiert. Nach einmaligem Waschen mit 200 $\mu$l PBS folgt die Behandlung der Ansätze mit Anti-BrdU-POD[1]-Lösung (1:100 in *"Antibody Dilution Solution"* für 90 min. Die Antikörperlösung wird entfernt, dreimal mit jeweils 200 $\mu$l *"Washing Solution"* gewaschen und 100 $\mu$l der Substratlösung aufgetragen. Der Verlauf der Farbreaktion wird beobachtet und gegebenenfalls durch Zugabe von 25 $\mu$l 1 M H$_2$SO$_4$ abgestoppt. Die Messung der Absorption erfolgt im ELISA-Reader "DynaTech MR5000" (Dynex Technologies, Großbritannien) bei einer Wellenlänge von 450 nm gegen eine Referenz von 690 nm [1]) Peroxidase.

**4. Ergebnisse:**

Hyperosmolare Bedingungen führen bei HaCaT-Zellen innerhalb von 8 h zu einer Abnahme der Proliferation um 40 - 50% gegenüber der isoosmolaren Kontrolle; nach 24 h beträgt der Anteil proliferierender Zellen noch 40 und nach 48 h 20% (vgl. Abb. 1).

Der Zusatz von Glycin, Ethylglycinat, führt bei humanen Keratinocyten, die unter hyperosmolaren Bedingungen kultiviert werden, zu einer signifikanten Steigerung der Proliferation gegenüber dem osmolytfreien, hyperosmolaren Kontrollansatz (0). Ein bei der Auswertung mitgeführter Ansatz unter isoosmolaren Bedingungen diente als Referenz für 100% Proliferation.

Ein maximal steigernder Effekt ist in einem Konzentrationsbereich von etwa $2,5 \times 10^{-2}$ bis $1 \times 10^{-4}$ M zu beobachten: die Proliferationsrate steigt durch Zugabe der Osmolyte auf mehr als das Doppelte im Vergleich zur hypertonischen Kontrolllösung an (vgl. Abb. 2 und 5).

Unter hyperosmolaren Bedingungen ist bei keiner Konzentration ein signifikanter Effekt durch Glycerin oder Inositol zu beobachten - die Proliferationsrate verbleibt auf dem Niveau des hyperosmolaren Kontrollansatzes ohne Zusatzstoff. Glycerin und Inositol weisen keinen schützenden Effekt gegenüber hyperosmolarem Stress auf (Abb.3 und 4). Hyperosmolarer Stress lässt sich auch durch die Zugabe von Sorbitol statt Kochsalz erzeugen. Auch gegen den auf diese Weise erzeugten Stress wirkt die Zugabe von erfindungsgemäßen Osmoprotektoren (Abb. 6).

Abb. 1

Abb. 2

Abb. 3

### Einfluss von Glycerin auf die Proliferation unter hyperosmolaren Bedingungen

Abb. 4

### Einfluss von Ethylglycinat auf die Proliferation unter hyperosmolaren Bedingungen

Abb. 5

## Einfluss von Sorbitol und organ. Osmolyte auf die Proliferation

Abb. 6

**5. Einfluss osmolaren Stresses auf die Apoptose von humanen Keratinozyten**

**5.1 Beschreibung des Zellsystems zur Untersuchung der Apoptose:**

Der Einfluss von hyperosmotischem Stress wird an der humanen Keratinozytenzelllinie HaCaT untersucht. Die Kultivierung der Zellen erfolgt im Inkubator bei 37°C, 5% (v/v) $CO_2$ und wasserdampfgesättigter Atmosphäre. Das entwickelte zelluläre Testsystem ist wie folgt aufgebaut: 40.000 Zellen/cm$^2$ werden in isoosmolarem Kulturmedium (Hank's Basalmedium, 0,002 M L-Glutamin, 5% (v/v) Fötales Kälberserum; Zusammensetzung des Basalmediums: siehe o. a. Patentschriften bzw. Erfindungsmeldung; Osmolarität: 0,3 osM) ausgesät und für mindestens 48 h inkubiert. Das Medium wird entfernt, der Zellrasen einmal mit phosphatgepufferter Salzlösung (PBS) gewaschen und für 24 h mit hyperosmolarem Kulturmedium (Osmolarität: 0,5 osM) ohne bzw. mit ausgewählten Osmolyten in den abgegebenen Konzentrationen inkubiert.

**5.2 Bestimmung der Apoptoserate in adhärent kultivierten Zellkulturen**

5.2.1 Direkte morphologische Auswertung DAPI gefärbter Zellen

[*vgl.:* Cohen, JJ. (1993) Apoptosis. Immunology Today, 14 (3): 126-130.]
DAPI (4',6-Diamidino-2-phenylindol, bezogen von Molecular Probes, Leiden, Niederlande) bindet spezifisch an zelluläre DNS und zeigt selbst bei geringen Farbstoffmengen eine hohe Fluoreszenzintensität. Im Fluoreszenzbild weisen vitale Zellen einen regelmäßigen, runden, homogen blau leuchtenden Zellkern auf. Die DNS-Fragmentierung apoptotischer Zellen führt zu einem unregelmäßig fragmentierten Kern mit hell leuchtenden Fragmenten, den sog. apoptotischen Körperchen. Hierdurch können unter dem Fluoreszenzmikroskop apoptotische von normalen Zellen eindeutig voneinander unterschieden werden.

Lösungen:

- PBS
- Formaldehyd Lösung
  4% (w/v) Formaldehyd in PBS, 4°C
- DAPI-Lösung
  60 nM DAPI in Methanol, p. A., lichtgeschützt bei 4°C mehrere Wochen haltbar.
  Anregungswellenlänge 340 nm
  Emissionswellenlänge 480 nm

Durchführung:

Der Kulturüberstand wird abgesaugt und der Zellrasen für 30 min mit 0,25 ml/cm$^2$ formaldehyd Lösung bei 4°C fixiert. Nach einmaligem Waschen mit 0,5 ml/cm$^2$ PBS wird die DNS der Zellen mit 0,25 ml/cm$^2$ DAPI-Lösung für 30 min bei 4°C gefärbt und die Zellen nochmals mit PBS gewaschen. Der Zellrasen wird mit 0,25 ml/cm$^2$ PBS überschichtet und unter dem Fluoreszenzmikroskop mit der angegebenen Anregungs- und Emissionswellenlänge ausgewertet.

**5.3 Definition der Apoptoserate**

Zur Quantifizierung der in einem Experiment erhaltenen Anzahl apoptotischer Zellen wird die sog. Apoptoserate verwendet. Diese ist folgendermaßen definiert:

$$A = \frac{Z_{apop}}{Z} \cdot 100$$

mit:

A = Apoptoserate [%]
$Z_{apop}$ = Anzahl apoptotischer Zellen
Z = Gesamtzellzahl

Für die Bestimmung der Apoptoserate werden drei unabhängig voneinander durchgeführte Experimente ausgewertet.
Unabhängig von der Wahl des verwendeten Objektivs bei der mikroskopischen Auswertung werden von jedem Ansatz mindestens 1000 Zellen in zufällig ausgewählten Gesichtsfeldern ausgezählt und die Apoptoserate berechnet.

**5.4 Ergebnisse**

Die Erhöhung der Osmolarität des Kulturmediums von 0,3 auf 0,5 osM führt zu einem Anstieg der Apoptoserate von 1,5 auf etwa 10%.
In Abb. 7 sind alle Einzelergebnisse bis auf das Taurin-Experiment zusammengefasst.
Die Zugabe der natürlichen organische Osmolyte Glycin, Betain, Taurin und Prolin (0,1 bzw. 10 mM) in das hyperosmolare Kulturmedium führt zu einem Rückgang der Apoptoserate um 2/3 bis zu ¾ gegenüber des hyperosmolaren Kontrollwertes ohne Osmolyt.
Die Wirkungsweise von Ethylglycinat wurde untersucht. In den entsprechenden Abbildungen dient der glycinhaltige Ansatz als Vergleichswert. Ethylglucruat zeigt ebenfalls einen apoptoseinhibierenden Einfluss.

**Abb. 7**

**6. Einfluss osmolaren Stresses auf die Expression des proinflammatorischen Cytokines Interleukin-6**

Die Bestimmung der Expression von IL-6 wird über den Anteil an IL-6 mRNA in den salzgestressten Zellkulturen ermittelt. Dies erfolgt durch quantitative RT-PCR unter Echtzeitbedingungen ("quantitative *RealTime* RT-PCR").

**6.1 Methoden**

**6.1.1 Zellkultivierung:**

Der Einfluss von hyperosmotischem Stress wird an der humanen Keratinozytenzelllinie HaCaT untersucht. Die Kultivierung der Zellen erfolgt im Inkubator bei 37°C, 5% (v/v) $CO_2$ und wasserdampfgesättigter Atmosphäre. Das entwickelte zelluläre Testsystem ist wie folgt aufgebaut: 40.000 Zellen/cm² werden in isoosmolarem Kulturmedium (Hanks' Basalmedium, 0,002 M L-Glutamin, 5% (v/v) Fötales Kälberserum; Zusammensetzung des Basalmediums: siehe o.a. Patentschriften bzw. Erfindungsmeldung; Osmolarität: 0,3 osM) ausgesät und für mindestens 48 h inkubiert. Das Medium wird entfernt, der Zellrasen einmal mit phosphatgepufferter Salzlösung (PBS) gewaschen und für 8 h mit hyperosmolarem Kulturmedium (Osmolarität: 0,5 osM) ohne bzw. mit ausgewählten Osmolyten in den angegebenen Konzentrationen inkubiert.

### 6.1.2 mRNA Isolierung:

Der Zellaufschluss mit anschließender Isolation der RNA erfolgt mit Hilfe des RNeasy-Kits der Fa. Qiagen, Hilden (Kat.Nr.: 75144), nach den vom Hersteller genannten Angaben.

### 6.1.3 Reverse Transkription:

Das Umschreiben der mRNA in cDNA erfolgt mit Hilfe des Omnisript-RT Kits der Fa. Qiagen, Hilden (Kat.Nr.: 205 111), nach den vom Hersteller genannten Angaben.

### 6.1.3 Quantitative *RealTime* RT-PCR:

*RealTime* PCR-Reaktionen werden mit dem PCR-Gerät "iCycler iQ" der Firma BioRad, München, durchgeführt. Die Durchführung erfolgt nach Herstellerangaben:

Die Zunahme an PCR-Produkt wird unter Verwendung des Fluoreszenzfarbstoffes SYBR-Green gemessen. Dieser Fluorophor hat die Eigenschaft, unspezifisch in Doppelstrang-DNA zu interkalieren. Somit wird die Zunahme an doppelsträngigem PCR-Produkt (in diesem Fall das IL-6 spezifische Amplicon) durch den Anstieg der Fluoreszenzintensität verfolgt.

Eine Differenzierung zwischen PCR-Produkt und Primer-Dimeren erfolgt durch Schmelzkurvenanalyse.

Mit Hilfe der delta-$C_t$-Methode wird die relative Expression von IL-6 unter den verschiedenen Versuchsbedingungen bestimmt.

Tabelle 3: PCR-Protokoll

|  |  |  | Zeit [min] |
|---|---|---|---|
| Cycle 1: | (1X) |  |  |
|  | Schritt 1: | 94,0°C | für 03:00 |
| Cycle 2: | (40X) |  |  |
|  | Schritt 1: | 94,0°C | für 00:45 |
|  | Schritt 2: | 58,0°C | für 00:30 |
|  | Schritt 3: | 72,0°C | für 00:45 |
| Cycle 3: | (1X) |  |  |
|  | Schritt 1: | 95,0°C | für 01:00 |
| Cycle 4: | (1X) |  |  |
|  | Schritt 1: | 55,0°C | für 01:00 |
| Cycle 5: | (80X) |  |  |
|  | Schritt 1: | 55,0°C | für 00:10 |

### 6.2 Ergebnisse

Unter dem Einfluss von hyperosmolarem Medium steigt der Anteil an Interleukin-6 mRNA auf das 260fache gegenüber der isoosmolaren Kontrolle an. Werden 10 mM Glycin in das hyperosmolare Medium hinzugegeben, so steigt die IL-6 Expression nur auf das 32-, bei Zugabe von 10 mM Ethylglycinat auf das 64fache an (siehe Abbildung 8). Demzufolge wirken diese Osmolyte antiinflammatorisch bzw. osmoprotektiv unter dem Einfluss von Salzstress in humanen Keratinozytenkulturen.

**Expression von Interleukin-6 unter Osmostress und Zugabe von Osmolyten**

Abb. 8

## 7. In-vitro Untersuchungen zur perkutanen Absorption von Wirkstoffen in die Schweinehaut

Der Hintergrund der Prüfung war die Bestimmung der perkutanen Absorption von ausgewählten Wirkstoffen in die Haut. Für die effektive Wirksamkeit einer Substanz ist es wichtig, in der entsprechenden Hautschicht eine ausreichende Wirkstoffkonzentration zu erreichen. Ein anerkanntes in vitro Modell stellt die Diffusionszelle nach Franz dar (Franz-type two-chamber glass cells; skin penetration system Modell LG 1084 LPC). Das Versuchsprotokoll wurde an die OECD Richtlinie 428 angelehnt. Für diesen Zweck wird eine bestimmte Menge der radioaktiv markierten Testsubstanz auf die exzidierten Schweinehaut topisch aufgetragen. Nach einer Versuchsdauer von 24 Stunden werden die Hautschichten (Stratum Corneum, Epidermis und die Dermis) analysiert. Dabei findet man eine gute Korrelation der Barriereeigenschaften der präparierten Schweinehaut mit den in vivo Verhältnissen.

In der "Leave-on" Formulierung wurden die Wirkstoffe jeweils in einer Konzentration von 2,5% in eine Basiscreme nach DAC (Deutscher Arzneimittel-Codex) eingearbeitet (Tabelle 4). Die aufgetragene Menge der Basiscreme betrug 32 mg/cm$^2$.

Tabelle 4:

**DAC Basiscreme (gem. INCI):**

| | |
|---|---|
| Glyceryl Stearate | 4,0 |
| Cetearyl Alcohol | 6,0 |
| Caprylic / Capric Triglyceride | 7,5 |
| Petrolatum | 25,5 |
| PEG-20 Glyceryl Stearate | 7,0 |
| Propylene Glycol | 10,0 |
| Aqua | 40,0 |

(fortgesetzt)

**DAC Basiscreme (gem. INCI):**

100,0

Wird die Aminosäure Glycin unmodfiziert auf die Haut aufgebracht, weist sie einen niedrigen Permeationskoeffizienten auf. *(A. Ruland and J. Kreuter, Transdermal , permeability and skin accumulation of amino acids; International Journal of Pharmaceutics; 72 (1991) 149-155)*.

Der Glycinethylester kann besser in die Haut penetrieren als Glycin. Überraschenderweise zeigte sich eine 10fache Anreicherung ("targeting") von Glycinethylester im Vergleich zum Glycin im Bereich der Epidermis (Tabelle 5 und 6). Taurin wurde verglichen mit Glycin in einer etwas größeren Menge in der Epidermis detektiert, jedoch in einer geringeren Menge als Glycinethylester (siehe Tabelle 5). Die Penetration von Glycinethylester in die Epidermis ist besonders günstig, da der Wirkstoff so besser an den Wirkort transportiert wird und so effektiver wirken kann. In der Epidermis befinden sich die Keratinozyten, die vor osmolarem Stress geschützt werden müssen. Durch Esterasen wird der Glycinethylester in der Haut gespalten und liegt wieder dann als der Wirkstoff Glycin vor. Es reicht also eine geringere Konzentration der Wirksubstanz in den erfindungsgemäßen Produkten im Vergleich zu Glycin aus, um eine entsprechende Wirkung am Wirkort zu erzielen.

Tabelle 5: In-vitro Hautpenetration (IHP) an der Schweinerückenhaut (Leave on)

| Substanzen (radioaktiv markiert $^{14}$C) | Dermis %* | Epidermis % | Stratum Corneum (SC) % |
|---|---|---|---|
| **Glycine ethyl ester Hydrochloride (Leave on)** | **2,22** | **1,29** | **1,17** |
| *Standardabweichung (SD)* | *0,58* | *0,47* | *0, 66* |
| **Glycin (Leave on)** | **0,85** | **0,13** | **0,39** |
| *SD* | *0,57* | *0,06* | *0,10* |
| **Taurin (Leave on)** | **0,90** | **0,20** | **0,28** |
| *SD* | *0,50* | *0,10* | *0, 06* |
| * IPH in Prozent über 24 Stunden; Eingesetzte Wirkstoffmenge in der Formulierung (AS) = 100% | | | |

Bei einer sogenannten "Rinse off" Anwendung ist die Kontaktzeit mit der Haut verkürzt. Die Substanzen wurden in einer Konzentration von jeweils 3% in eine milde Tensidformulierung (Fa® Wellness System Duschgel, Spring Flower SPA mit Meeresmineralien, Schwarzkopf & Henkel) eingearbeitet und für 30 Minuten auf der Haut appliziert. Die Applikationsmenge betrug 30 mg/cm². Dieses Testdesign soll eine häufigere aber kürzere Anwendung simulieren. Diese häufigere und kürzere Anwendungszeit lässt sich aufgrund der eingeschränkten Haltbarkeit der Schweinehaut nicht über mehrere Tage ausdehnen.

Tabelle 6: In-vitro Hautpenetration (IHP) an der Schweinerückenhaut (rinse-off)

| Substanzen (radioaktiv markiert $^{14}$C) | Konzentration Aktivsubstanz (Gew.-%) (AS) | Dermis % | Epidermis % | SC % |
|---|---|---|---|---|
| **Glycinethylester (Hydrochlorid) (Rinse off)** | 3 | **0,16** | **0,08** | **0,20** |
| ***SD*** | | *0,22* | *0,08* | *0,10* |
| **Glycin (Rinse off)** | 3 | **0,04** | **0,00** | **0,01** |
| ***SD*** | | *0,08* | *0,00* | *0,01* |
| * IPH in Prozent über 24 Stunden; Eingesetzte Wirkstoffmenge = 100% | | | | |

Durch die kurze Kontaktzeit der Wirksubstanz mit der Haut konnte in der Epidermis kein Glycin und im Stratum Corneum und in der Dermis nur eine sehr geringe Menge an Glycin gefunden werden. Glycinethylester konnte auch bei dieser Applikationsart besser in die Haut penetrieren als Glycin.

**Patentansprüche**

1. Verwendung von Ethylglycinat zum Schutz der Haut und/oder der Hautanhangsgebilde in einer kosmetischen Anwendung.

2. Verwendung nach Anspruch 1, **dadurch gekennzeichnet, dass** das Ethylglycinat zum Schutz der Haut und/oder der Hautzellen vor osmotischem Stress eingesetzt wird.

3. Ethylglycinat zur Anwendung in einem Verfahren zum Schutz der Haut und/oder der Hautanhangsgebilde vor Entzündungen.

4. Ethylglycinat nach Anspruch 3, **dadurch gekennzeichnet, dass** das Ethylglycinat zum Schutz vor Entzündungen eingesetzt wird, die durch osmotischen Stress verursacht werden.

5. Verwendung nach einem der vorstehenden Ansprüche, **dadurch gekennzeichnet, dass** das Ethylglycinat zum Schutz der Haut vor UV-Stress eingesetzt wird.

6. Verwendung nach einem der vorstehenden Ansprüche, **dadurch gekennzeichnet, dass** das Ethylglycinat zum Schutz der Haut vor Apoptose eingesetzt wird.

7. Verwendung nach Anspruch 5 oder 6, **dadurch gekennzeichnet, dass** das Ethylglycinat zum Schutz der Haut vor Apoptose eingesetzt wird, die durch UV-Stress und/oder osmotischen Stress verursacht wird.

8. Verwendung nach einem der vorstehenden Ansprüche, **dadurch gekennzeichnet, dass** das Ethylglycinat in Zubereitungen zwischen 0,005 bis 15 Gew.-% enthalten ist.

9. Verwendung nach einem der vorstehenden Ansprüche, **dadurch gekennzeichnet, dass** das Ethylglycinat in einer kosmetischen Zubereitung vorliegt.

10. Verwendung nach Anspruch 9, **dadurch gekennzeichnet, dass** die kosmetischen Zubereitungen ausgewählt sind aus Haar- und/oder Haut- bzw. Körper- und/oder Mund- bzw. Zahnpflegeprodukten.

11. Verwendung nach Anspruch 10, **dadurch gekennzeichnet, dass** die kosmetischen Zubereitungen ausgewählt sind aus Zahncremes, -gelen, Mundwässern, Mundspülungen, Hautpflegecremes, After-sun-Produkten, tensidischen Produkten wie Duschbädern und/oder Shampoos, Haarnachbehandlungsmitteln, wie Kuren oder Spülungen, oder Haarfärbemitteln.

12. Verwendung nach einem der vorstehenden Ansprüche, **dadurch gekennzeichnet, dass** das Ethylgylcinat zur Applikation auf feste Träger aufgetragen wird, insbesondere Windeln, Binden, Slip- oder Inkontinenzeinlagen, Kosmetiktüchern für Reinigung und Pflege, Verbände oder Pflaster.

13. Verwendung nach Anspruch 12, **dadurch gekennzeichnet, dass** das Ethylglycinat in kosmetischen Zubereitungen auf den festen Träger aufgetragen wird.

14. Verwendung von Ethylglycinat zur Herstellung einer pharmazeutischen Zubereitung zum Schutz der Haut und/oder der Hautanhangsgebilde.

15. Verwendung nach Anspruch 14, **dadurch gekennzeichnet, dass** das Ethylglycinat zum Schutz der Haut und/oder der Hautzellen vor osmotischem Stress eingesetzt wird.

16. Verwendung nach Anspruch 14 oder 15, **dadurch gekennzeichnet, dass** das Ethylglycinat zum Schutz der Haut vor Entzündungen eingesetzt wird.

17. Verwendung nach einem der Ansprüche 14 bis 16, **dadurch gekennzeichnet, dass** das Ethylglycinat zum Schutz der Haut vor Entzündungen eingesetzt wird, die durch osmotischen Stress verursacht werden.

18. Verwendung nach einem der Ansprüche 14 bis 17, **dadurch gekennzeichnet, dass** das Ethylglycinat zum Schutz der Haut vor UV-Stress eingesetzt wird.

**19.** Verwendung nach einem der Ansprüche 14 bis 18, **dadurch gekennzeichnet, dass** das Ethylglycinat zum Schutz der Haut vor Apoptose eingesetzt wird.

**20.** Verwendung nach einem der Ansprüche 14 bis 19, **dadurch gekennzeichnet, dass** das Ethylglycinat zum Schutz der Haut vor Apoptose eingesetzt wird, die durch UV-Stress und/oder osmotischen Stress verursacht wird.

**21.** Verwendung nach einem der Ansprüche 14 bis 20, **dadurch gekennzeichnet, dass** das Ethylglycinat in Zubereitungen zwischen 0,005 bis 15 Gew.% enthalten ist.

**22.** Verwendung von Ethylglycinat zur Herstellung einer pharmazeutischen Zubereitung zur Wundheilung und/oder Behandlung von atopischer Dermatitis, Psoriasis oder Windelekzem.

**23.** Verwendung nach einem der Ansprüche 14 bis 22, **dadurch gekennzeichnet, dass** das Ethylgylcinat zur Applikation auf feste Träger aufgetragen wird, insbesondere Windeln, Binden, Slip- oder Inkontinenzeinlagen, Kosmetiktüchern für Reinigung und Pflege, Verbände oder Pflaster.

**24.** Verwendung nach Anspruch 23, **dadurch gekennzeichnet, dass** das Ethylglycinat in pharmazeutischen Zubereitungen auf den festen Träger aufgetragen wird.

**Claims**

**1.** Use of ethyl glycinate to protect the skin and/or skin appendages in a cosmetic application.

**2.** Use according to claim 1, **characterized in that** the ethyl glycinate is used to protect the skin and/or skin cells from osmotic stress.

**3.** Ethyl glycinate for use in a method for protecting the skin and/or skin appendages from inflammation.

**4.** Ethyl glycinate according to claim 3, **characterized in that** the ethyl glycinate is used to provide protection from inflammation caused by osmotic stress.

**5.** Use according to any one of the preceding claims, **characterized in that** the ethyl glycinate is used to protect the skin from UV stress.

**6.** Use according to any one of the preceding claims, **characterized in that** the ethyl glycinate is used to protect the skin from apoptosis.

**7.** Use according to claim 5 or claim 6, **characterized in that** the ethyl glycinate is used to protect the skin from apoptosis caused by UV stress and/or osmotic stress.

**8.** Use according to any one of the preceding claims, **characterized in that** the ethyl glycinate is contained in preparations in a quantity of between 0.005 and 15 wt.%.

**9.** Use according to any one of the preceding claims, **characterized in that** the ethyl glycinate is present in a cosmetic preparation.

**10.** Use according to claim 9, **characterized in that** the cosmetic preparations are selected from hair and/or skin or body and/or oral or dental care products.

**11.** Use according to claim 10, **characterized in that** the cosmetic preparations are selected from toothpastes, tooth gels, mouth washes, mouth rinses, skin care creams, aftersun products, surfactant products such as shower foams and/or shampoos, hair aftertreatment preparations, such as masks or rinses, or hair dyes.

**12.** Use according to any one of the preceding claims, **characterized in that** the ethyl glycinate is applied, for administration, to solid supports, in particular nappies, sanitary towels, panty liners or incontinence pads, facial tissues for cleansing and hygiene, dressings or plasters.

13. Use according to claim 12, **characterized in that** the ethyl glycinate is applied to the solid support in cosmetic preparations.

14. Use of ethyl glycinate for producing a pharmaceutical preparation for protecting the skin and/or skin appendages.

15. Use according to claim 14, **characterized in that** the ethyl glycinate is used to protect the skin and/or skin cells from osmotic stress.

16. Use according to claim 14 or claim 15, **characterized in that** the ethyl glycinate is used to protect the skin from inflammation.

17. Use according to any one of claims 14 to 16, **characterized in that** the ethyl glycinate is used to protect the skin from inflammation caused by osmotic stress.

18. Use according to any one of claims 14 to 17, **characterized in that** the ethyl glycinate is used to protect the skin from UV stress.

19. Use according to any one of claims 14 to 18, **characterized in that** the ethyl glycinate is used to protect the skin from apoptosis.

20. Use according to any one of claims 14 to 19, **characterized in that** the ethyl glycinate is used to protect the skin from apoptosis caused by UV stress and/or osmotic stress.

21. Use according to any one of claims 14 to 20, **characterized in that** the ethyl glycinate is contained in preparations in a quantity of between 0.005 and 15 wt.%.

22. Use of ethyl glycinate for producing a pharmaceutical preparation for wound healing and/or for treating atopical dermatitis, psoriasis or nappy rash.

23. Use according to any one of claims 14 to 22, **characterized in that** the ethyl glycinate is applied, for administration, to solid supports, in particular nappies, sanitary towels, panty liners or incontinence pads, facial tissues for cleansing and hygiene, dressings or plasters.

24. Use according to claim 23, **characterized in that** the ethyl glycinate is applied to the solid support in pharmaceutical preparations.


**Revendications**

1. Utilisation de glycinate d'éthyle pour la protection de la peau et/ou des phanères dans une application cosmétique.

2. Utilisation selon la revendication 1, **caractérisée en ce qu'**on met en oeuvre le glycinate d'éthyle pour la protection de la peau et/ou des cellules cutanées contre le stress osmotique.

3. Glycinate d'éthyle pour l'application dans un procédé pour la protection de la peau et/ou des phanères contre des inflammations.

4. Glycinate d'éthyle selon la revendication 3, **caractérisé en ce qu'**on met en oeuvre le glycinate d'éthyle pour la protection contre des inflammations qui sont provoquées par le stress osmotique.

5. Utilisation selon l'une quelconque des revendications précédentes, **caractérisée en ce qu'**on met en oeuvre le glycinate d'éthyle pour la protection de la peau contre le stress UV.

6. Utilisation selon l'une quelconque des revendications précédentes, **caractérisée en ce qu'**on met en oeuvre le glycinate d'éthyle pour la protection de la peau contre l'apoptose.

7. Utilisation selon la revendication 5 ou 6, **caractérisée en ce qu'**on met en oeuvre le glycinate d'éthyle pour la protection de la peau contre l'apoptose qui est provoquée par le stress UV et/ou par le stress osmotique.

8. Utilisation selon l'une quelconque des revendications précédentes, **caractérisée en ce que** le glycinate d'éthyle est contenu dans des préparations entre 0,005 et 15 % en poids.

9. Utilisation selon l'une quelconque des revendications précédentes, **caractérisée en ce que** le glycinate d'éthyle est présent dans une préparation cosmétique.

10. Utilisation selon la revendication 9, **caractérisée en ce que** les préparations cosmétiques sont choisies parmi des produits d'entretien pour les cheveux et/ou pour la peau respectivement pour le corps et/ou pour la bouche respectivement pour les dents.

11. Utilisation selon la revendication 10, **caractérisée en ce que** les préparations cosmétiques sont choisies parmi des crèmes dentaires, des gels dentaires, des collutoires, des bains de bouches, des crèmes de soin pour la peau, des produits après soleil, des produits tensioactifs tels que des produits pour la douche et pour le bain et/ou des shampooings, des agents de traitement ultérieur des cheveux tels que des cures ou des rinçages, ou encore des teintures capillaires.

12. Utilisation selon l'une quelconque des revendications précédentes, **caractérisée en ce qu'**on met le glycinate d'éthyle pour l'application sur des supports solides, en particulier des couches, des bandes, des protège-slips ou des bandelettes pour l'incontinence, des serviettes à usage cosmétique pour le nettoyage et les soins, des pansements ou des emplâtres.

13. Utilisation selon la revendication 12, **caractérisée en ce que** l'on applique le glycinate d'éthyle dans des préparations cosmétiques, sur le support solide.

14. Utilisation de glycinate d'éthyle pour la production d'une préparation pharmaceutique destinée à la protection de la peau et/ou des phanères.

15. Utilisation selon la revendication 14, **caractérisée en ce que** le glycinate d'éthyle est mis en oeuvre pour la protection de la peau et/ou des cellules cutanées contre le stress osmotique.

16. Utilisation selon la revendication 14 ou 15, **caractérisée en ce que** le glycinate d'éthyle est mis en oeuvre pour la protection de la peau contre les inflammations.

17. Utilisation selon l'une quelconque des revendications 14 à 16, **caractérisée en ce que** le glycinate d'éthyle est mis en oeuvre pour la protection de la peau contre des inflammations qui sont provoquées par le stress osmotique.

18. Utilisation selon l'une quelconque des revendications 14 à 17, **caractérisée en ce que** le glycinate d'éthyle est mis en oeuvre pour la protection de la peau contre le stress UV.

19. Utilisation selon l'une quelconque des revendications 14 à 18, **caractérisée en ce que** le glycinate d'éthyle est mis en oeuvre pour la protection de la peau contre l'apoptose.

20. Utilisation selon l'une quelconque des revendications 14 à 19, **caractérisée en ce que** le glycinate d'éthyle est mis en oeuvre pour la protection de la peau contre l'apoptose qui est provoquée par le stress UV et/ou par le stress osmotique.

21. Utilisation selon l'une quelconque des revendications 14 à 20, **caractérisée en ce que** le glycinate d'éthyle est contenu dans des préparations entre 0,005 et 15 % en poids.

22. Utilisation du glycinate d'éthyle pour la production d'une préparation pharmaceutique destinée à la cicatrisation et/ou au traitement de la dermatite atopique, du psoriasis ou de l'eczéma du nourrisson.

23. Utilisation selon l'une quelconque des revendications 14 à 22, **caractérisée en ce qu'**on met le glycinate d'éthyle pour l'application sur des supports solides, en particulier des couches, des bandes, des protège-slips ou des bandelettes pour l'incontinence, des serviettes à usage cosmétique pour le nettoyage et les soins, des pansements ou des emplâtres.

24. Utilisation selon la revendication 23, **caractérisée en ce que** l'on applique le glycinate d'éthyle dans des préparations pharmaceutiques, sur le support solide.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 0004870 A
- WO 0119330 A
- DE 2703964
- EP 124091 A
- WO 0101932 A
- DE 19740092
- DE 1165574
- DE 2024051
- FR 2252840 A

- EP 0693471 B1
- EP 0818450 A1
- EP 0694521 B1
- DE 19712033 A1
- US 4153680 A
- US 3538230 A
- DE OS2522586 A
- DE OS3114493 A

**In der Beschreibung aufgeführte Nicht-Patentliteratur**

- **TODD et al.** *Cosm.Toil.,* 1976, vol. 91, 27
- **R.LOCHHEAD.** *Cosm.Toil.,* 1993, vol. 108, 95
- Deutschen Forschungsgemeinschaft. Verlag Chemie, 1984, 81-106
- Ullmann, Enzyclopädie der technischen Chemie. 1974, vol. 7, 298
- **KH. SCHRADER.** Grundlagen und Rezepturen der Kosmetika. Hüthig Buch Verlag, 1989
- **W. UMBACH.** Kosmetik: Entwicklung, Herstellung und Anwendung kosmetischer Mittel. Thieme Verlag, 1995
- *J.Soc.Cosm.Chem.,* 1973, vol. 24, 281

- *J.Pharm.Pharmacol.,* 1975, vol. 26, 531
- **BOUKAMP, P. ; PETRUSSEVSKA, R. T. ; BREITKREUTZ, D. ; HORNUNG, J. ; MARKHAM, A. ; FUSENIG, N.E.** Normal keratinization in a spontaneously immortalized aneuploid human keratinocyte cell line. *J. Cell Biol.,* 1988, vol. 106 (3), 761-771
- **COHEN, JJ.** *Apoptosis. Immunology Today,* 1993, vol. 14 (3), 126-130
- **A. RULAND ; J. KREUTER.** Transdermal , permeability and skin accumulation of amino acids. *International Journal of Pharmaceutics,* 1991, vol. 72, 149-155